# EUROPÄISCHE PATENTANMELDUNG

(11) **EP 3 178 830 A1**
(43) Veröffentlichungstag der Anmeldung: **14.06.2017**
(21) Anmeldenummer: 16201660.4
(22) Anmeldetag: 01.12.2016
(51) Int. Cl.: C07F 15/00, C07B 41/06, C07C 391/02, B01J 31/28, C07F 11/00, C07C 45/50

(54) **KOMPLEXE VON DIPHENYLSELENOXIDEN, DEREN VERWENDUNG UND KATALYSEVERFAHREN**

(30) Priorität: 07.12.2015 EP 15198150; 08.04.2016 DE 102016205888
(71) Anmelder: Evonik Degussa GmbH, 45128 Essen (DE)
(72) Erfinder: Dyballa, Katrin Marie, 45657 Recklinghausen (DE); Franke, Robert, 45772 Marl (DE); Selent, Detlef, 18059 Rostock (DE); Börner, Armin, 18059 Rostock (DE); Weilbeer, Claudia, 06406 Bernburg (DE)

(57) **Zusammenfassung**

Neue Komplexe von Diphenylselenoxiden sowie deren Verwendung und Verfahren in denen die Komplexe eingesetzt werden.

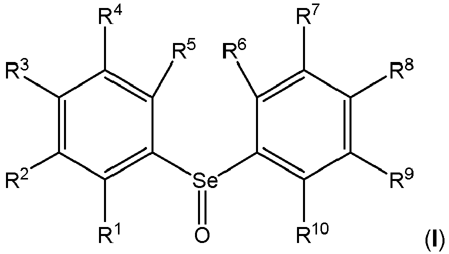

## Beschreibung

Neue Komplexe von Diphenylselenoxiden sowie deren Verwendung und Verfahren in denen die Komplexe eingesetzt werden.

Eine Syntheseroute zur Herstellung von Diphenylseleniden wie im experimentellen Teil offenbart, wird auch in der europäischen Patentanmeldung EP15168377.8 beschrieben. Die erhaltenen Diphenylselenide können anschließend zu Diphenylselenoxiden oxidiert werden.

In der Hydroformylierung werden in der Regel Mono- und Bisphosphite eingesetzt, die oftmals aus Biphenol-Bausteinen aufgebaut sind. Die Entwicklung neuer Liganden ist häufig durch die zur Verfügung stehenden Ligandenbausteine limitiert. So stellen Diphenylselenoxide eine hochinteressante Verbindungsklasse dar, die sich auch als Ligand in Komplexen bzw. als Ligandenbausteine für die Herstellung von Liganden eignet.

Aufgabe der Erfindung war es, eine weitere gänzlich neue Substanzklasse an Ligandenbausteinen und Katalysatoren herzustellen, um das Feld der verfügbaren Liganden für die jeweiligen spezifischen Komplexe in der Katalyse zu erweitern. Des Weiteren bestand die Aufgabe Liganden für Rhodium-Hydroformylierungskatalysatoren bereitzustellen oder herzustellen. Daher bestand auch die Aufgabe neue Ligandenbausteine bereitzustellen, die als Zwischenprodukt zur Herstellung von Liganden dienen. Eine zusätzliche Aufgabe bestand darin Liganden oder Ligandenbausteine für eine gezielte Steuerung der Katalyse bereitzustellen.

Gelöst werden die Aufgaben durch Diphenylselenoxide die am Selen zweifach Arylfunktionalisiert sind. Ferner werden die Aufgaben gelöst durch Komplexe enthaltend diese Diphenylselenoxide. Die erfindungsgemäßen Diphenylselenoxide sind ausgewählt aus unsubstituierten oder substituierten Diphenylselenoxiden der Struktur I, sowie vorzugsweise der Strukturen **Ia**, **Ib**, **Ib***, **Ic** und **Id**, als auch aus Gemischen enthaltend mindestens zwei der vorgenannten Strukturen.

Gegenstand der Erfindung ist ein Komplex umfassend mindestens ein Diphenylselenoxid, welches eine allgemeine Struktur (I) aufweist 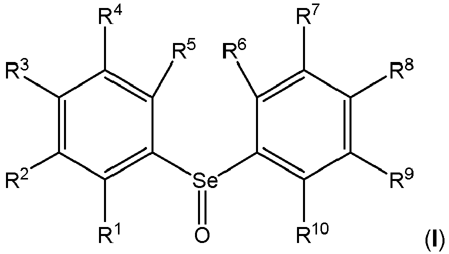 mit R², R³, R⁴, R⁵, R⁶, R⁷, R⁸ und R⁹, die jeweils unabhängig ausgewählt sein können aus - H, -(C₁-C₁₂)-Alkyl, -Halogen, mit Alkyl linear, verzweigt oder cyclisch, wobei die Alkylgruppen jeweils unabhängig unsubstituiert sind oder optional kann ein vorgenanntes Alkyl mit mindesten einer -(C₃-C₁₂)-Cycloalkylgruppe substituiert sein, wobei R¹ und R¹⁰ jeweils unabhängig ausgewählt sind aus -H, -(C₁-C₁₂)-Alkyl und/oder -Halogen, mit Alkyl linear, verzweigt oder cyclisch und mindestens ein Metallatom ausgewählt aus Rh, Ru, Co, Ir. Erfindungsgemäss kann es bevorzugt sein, wenn alle Alkylgruppen unsubstituiert sind.

Besonders bevorzugte Komplexe umfassen als mindestens ein Metallatom Rh, Ir, Ru, wobei Rh besonders bevorzugt ist.

Die Begriffe Diphenylselenoxid und Olefin werden in dieser Anmeldung als Gattungsbegriff verwendet und umfassen jeweils somit neben den unsubstituierten auch die substituierten Verbindungen.

In einer Ausführungsform umfasst der Komplex mindestens ein Diphenylselenid, welches eine allgemeine Struktur (**Ia**) aufweist 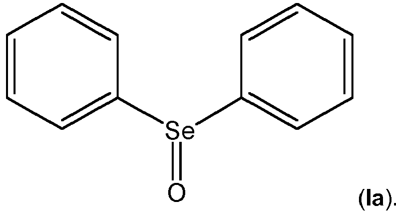 In einer Ausführungsform umfasst der Komplex mindestens ein Diphenylselenid, welches eine allgemeine Struktur **(Ib)** aufweist 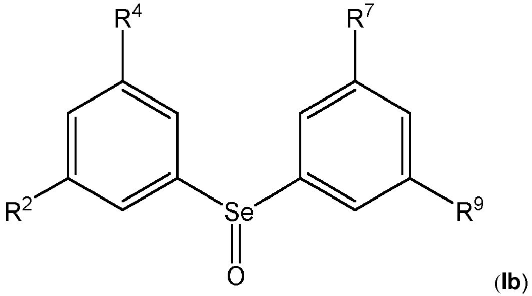 wobei in der Struktur **Ib** R², R⁴, R⁷ und R⁹ jeweils unabhängig ausgewählt sind aus -(C₁-C₁₂)-Alkyl.

In einer Ausführungsform umfasst der Komplex mindestens ein Diphenylselenid, welches eine allgemeine Struktur (**Ib***) aufweist

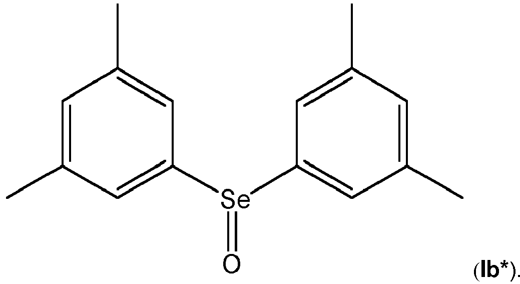

In einer Ausführungsform umfasst der Komplex mindestens ein Diphenylselenid, welches eine allgemeine Struktur (**Ic**) aufweist 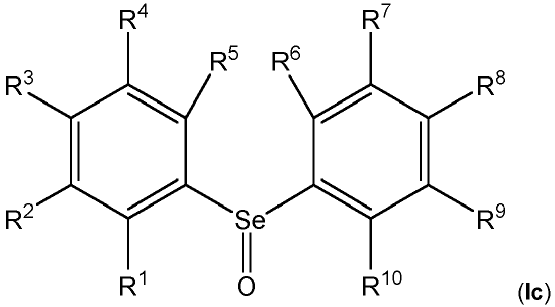 mit R³, R⁵, R⁶ und R⁸ jeweils -H und R², R⁴, R⁷ und R⁹ jeweils unabhängig ausgewählt aus -(C₁-C₁₂)-Alkyl, mit Alkyl linear, verzweigt oder cyclisch, mit R¹, R¹⁰ jeweils unabhängig ausgewählt aus -H, -(C₁-C₁₂)-Alkyl und -Halogen, mit Alkyl linear, verzweigt oder cyclisch.

In einer Ausführungsform umfasst der Komplex mindestens ein Diphenylselenid, welches eine allgemeine Struktur **(Id)** aufweist 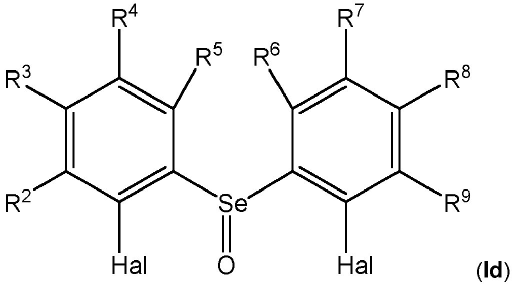 mit R², R³, R⁴, R⁵, R⁶, R⁷, R⁸ und R⁹ jeweils unabhängig ausgewählt aus -H, -(C₁-C₁₂)-Alkyl, und -Halogen.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt um -(C₁-C₆)-Alkylgruppen. Beispiele für -(C₁-C₁₂)-Alkylgruppen sind, insbesondere Methyl, Ethyl, Propyl, Isopropyl, n-Butyl, iso-Butyl, sec.-Butyl, *tert*.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Diemthylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, n-Heptyl, 2-Heptyl, 3-Heptyl, 2-Ethylpentyl, 1-Propylbutyl, n-Octyl, 2-Ethylhexyl, 2-Propylheptyl, Nonyl-, Decyl.

Halogen (Hal) umfasst Chlor, Brom, Fluor und Jod, wobei Chlor und Fluor besonders bevorzugt sind.

Die Erläuterungen zum Ausdruck -(C₁-C₁₂)-Alkyl gelten für alle Alkylgruppen.

Gemäß einer Ausführungsvariante können R², R³, R⁴, R⁵, R⁶, R⁷, R⁸ und R⁹ die jeweils unabhängig einem substituierten Alkyl mit mindesten einer -(C₃-C₁₂)-Cycloalkylgruppe entsprechen können, als -(C₃-C₁₂)-Cycloalkyl im Sinne der vorliegenden Erfindung umfassen, mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl. Ein Beispiel für ein substituiertes Cycloalkyl wäre Menthyl.

Besonders bevorzugte Komplexe umfassen mindestens ein Rhodium-Atom sowie mindestens ein Diphenylselenoxid der Struktur **Ia**, **Ib**, **Ib***, **Ic** und/oder **Id**, wobei vorzugsweise in der Struktur **Ic** R¹ und R¹⁰ jeweils unabhängig ausgewählt sind aus -H oder -(C₁-C₁₂)-Alkyl, mit Alkyl linear, verzweigt oder cyclisch, bevorzugt sind in der Struktur **Ic** R¹ und R¹⁰ jeweils gleich und ausgewählt aus -H oder -(C₁-C₆)-Alkyl, mit Alkyl verzweigt oder cyclisch.

Besonders bevorzugte Komplexe umfassen mindestens ein Ruthenium-Atom sowie mindestens ein Diphenylselenoxid der Struktur **Ia**, **Ib**, **Ib***, **Ic** und/oder **Id**, wobei vorzugsweise in der Struktur **Ic** R¹ und R¹⁰ jeweils unabhängig ausgewählt sind aus -H oder -(C₁-C₁₂)-Alkyl, mit Alkyl linear, verzweigt oder cyclisch, bevorzugt sind in der Struktur **Ic** R¹ und R¹⁰ jeweils gleich und ausgewählt aus -H oder -(C₁-C₆)-Alkyl, mit Alkyl verzweigt oder cyclisch.

Besonders bevorzugte Komplexe umfassen mindestens ein Kobalt-Atom sowie mindestens ein Diphenylselenoxid der Struktur **Ia**, **Ib**, **Ib***, **Ic** und/oder **Id**, wobei vorzugsweise in der Struktur **Ic** R¹ und R¹⁰ jeweils unabhängig ausgewählt sind aus -H oder -(C₁-C₁₂)-Alkyl, mit Alkyl linear, verzweigt oder cyclisch, bevorzugt sind in der Struktur **Ic** R¹ und R¹⁰ jeweils gleich und ausgewählt aus -H oder -(C₁-C₆)-Alkyl, mit Alkyl verzweigt oder cyclisch.

Besonders bevorzugte Komplexe umfassen mindestens ein Iridium-Atom sowie mindestens ein Diphenylselenoxid der Struktur **Ia**, **Ib, Ib***, **Ic** und/oder **Id**, wobei vorzugsweise in der Struktur **Ic** R¹ und R¹⁰ jeweils unabhängig ausgewählt sind aus -H oder -(C₁-C₁₂)-Alkyl, mit Alkyl linear, verzweigt oder cyclisch, bevorzugt sind in der Struktur **Ic** R¹ und R¹⁰ jeweils gleich und ausgewählt aus -H oder -(C₁-C₆)-Alkyl, mit Alkyl verzweigt oder cyclisch.

Besonders bevorzugt sind R², R⁴, R⁷, R⁹ in der Struktur **I** jeweils unabhängig voneinander ausgewählt aus -H, -(C₁-C₁₂)-Alkyl und -Halogen und R¹, R³, R⁵, R⁶, R⁸, R¹⁰ sind -H.

Besonders bevorzugt sind Diphenylselenoxide der Struktur **I** mit R¹ und R¹⁰ und vorzugsweise beide gleich -H oder -(C₁-C₁₂)-Alkyl, mit Alkyl linear, verzweigt oder cyclisch oder R¹ gleich -H und R¹⁰ gleich -(C₁-C₁₂)-Alkyl, mit Alkyl linear, verzweigt oder cyclisch. Vorzugsweise ist Alkyl ausgewählt aus Methyl, Ethyl, Isopropyl, *tert*.-Butyl, iso-Pentyl, 1,1-Dimethylpropyl. Vorzugsweise sind R², R³, R⁴, R⁵, R⁶, R⁷, R⁸, R⁹ gleich -H.

Besonders bevorzugt sind Diphenylselenoxide der Struktur **I,** mit R¹ gleich -H und R¹⁰ gleich -Halogen. Mit R², R³, R⁴, R⁵, R⁶, R⁷, R⁸, R⁹ unabhängig ausgewählt aus -H, -(C₁-C₁₂)-Alkyl, mit Alkyl linear, verzweigt oder cyclisch.

Weitere bevorzugte Diphenylselenoxide weisen als R², R⁴, R⁷ und R⁹ gleich Methyl auf mit R¹ und R¹⁰ beide gleich -Halogen, -H oder -(C₁-C₆)-Alkyl, insbesondere Methyl, Ethyl, Isopropyl, *tert*.-Butyl, iso-Pentyl, 1,1-Dimethylpropyl. Vorzugsweise sind R³, R⁵, R⁶, R⁸ gleich -H.

Weitere bevorzugte Diphenylselenoxide weisen als R², R⁴, R⁵, R⁶, R⁷ und R⁹ gleich Methyl auf mit R¹ und R¹⁰ beide gleich -Halogen, -H oder -(C₁-C₆)-Alkyl, insbesondere Methyl, Ethyl, Isopropyl, *tert*.-Butyl, iso-Pentyl, 1,1-Dimethylpropyl. Vorzugsweise sind R³ und R⁸ gleich -H.

Weitere bevorzugte Diphenylselenoxide weisen als R² und R⁹ Isopropyl und als R⁴, R⁵, R⁶ und R⁷ gleich Methyl auf mit R¹ und R¹⁰ beide gleich -Halogen, -H oder -(C₁-C₆)-Alkyl, insbesondere Methyl, Ethyl, Isopropyl, *tert*.-Butyl, iso-Pentyl, 1,1-Dimethylpropyl. Vorzugsweise sind R³ und R⁸ gleich -H.

Weitere bevorzugte Diphenylselenoxide weisen als R² und R⁹ Isopropyl und als R⁵ und R⁶ gleich Methyl auf, R⁴ und R⁷ sind gleich -Halogen, insbesondere sind R⁴ und R⁷ beide gleich -Chlor, mit R¹ und R¹⁰ beide gleich -Halogen, -H oder -(C₁-C₆)-Alkyl, insbesondere Methyl, Ethyl, Isopropyl, *tert*.-Butyl, iso-Pentyl, 1,1-Dimethylpropyl. Vorzugsweise sind R³ und R⁸ gleich -H.

Weitere bevorzugte Diphenylselenoxide weisen als R² und R⁹ Methyl und als R⁴ und R⁷ gleich -Halogen auf, insbesondere sind R⁴ und R⁷ beide gleich -Chlor, mit R¹ und R¹⁰ beide gleich -Halogen, -H oder -(C₁-C₆)-Alkyl, insbesondere Methyl, Ethyl, Isopropyl, *tert*.-Butyl, iso-Pentyl, 1,1-Dimethylpropyl. Vorzugsweise sind R³, R⁵, R⁶, R⁸ gleich -H.

Weitere bevorzugte Diphenylselenoxide weisen als R⁴ und R⁷ Methyl und als R² und R⁹ gleich -Halogen auf, insbesondere sind R² und R⁹ beide gleich -Brom, mit R¹ und R¹⁰ beide gleich -Halogen, -H oder -(C₁-C₆)-Alkyl, insbesondere Methyl, Ethyl, Isopropyl, *tert*.-Butyl, iso-Pentyl, 1,1-Dimethylpropyl. Vorzugsweise sind R³, R⁵, R⁶, R⁸ gleich -H.

Weitere bevorzugte Diphenylselenoxide weisen als R⁴ und R⁷ *tert*.-Butyl und als R² und R⁹ gleich Methyl auf mit R¹ und R¹⁰ beide gleich -Halogen, -H oder -(C₁-C₆)-Alkyl, insbesondere Methyl, Ethyl, Isopropyl, *tert*.-Butyl, iso-Pentyl, 1,1-Dimethylpropyl. Vorzugsweise sind R³, R⁵, R⁶, R⁸ gleich -H.

Weitere bevorzugte Diphenylselenoxide weisen als R², R⁴, R⁷ und R⁹ gleich *tert*.-Butyl auf mit R¹ und R¹⁰ beide gleich -Halogen, -H oder -(C₁-C₆)-Alkyl, insbesondere Methyl, Ethyl, Isopropyl, *tert*.-Butyl, iso-Pentyl, 1,1-Dimethylpropyl. Vorzugsweise sind R³, R⁵, R⁶, R⁸ gleich -H.

Weitere bevorzugte Diphenylselenoxide weisen als R⁴ und R⁷ Ethyl und als R² und R⁹ gleich *tert*.-Butyl auf mit R¹ und R¹⁰ beide gleich -Halogen, -H oder -(C₁-C₆)-Alkyl, insbesondere Methyl, Ethyl, Isopropyl, *tert*.-Butyl, iso-Pentyl, 1,1-Dimethylpropyl. Vorzugsweise sind R³, R⁵, R⁶, R⁸ gleich -H.

Weitere bevorzugte Diphenylselenoxide weisen als R⁴ und R⁷ Ethyl und als R² und R⁹ gleich iso-Pentyl auf mit R¹ und R¹⁰ beide gleich -Halogen, -H oder -(C₁-C₆)-Alkyl, insbesondere Methyl, Ethyl, Isopropyl, *tert*.-Butyl, iso-Pentyl, 1,1-Dimethylpropyl. Vorzugsweise sind R³, R⁵, R⁶, R⁸ gleich -H.

Weitere bevorzugte Diphenylselenoxide weisen als R², R⁴, R⁷ und R⁹ gleich iso-Pentyl auf mit R¹ und R¹⁰ beide gleich -Halogen, -H oder -(C₁-C₆)-Alkyl, insbesondere Methyl, Ethyl, Isopropyl, *tert*.-Butyl, iso-Pentyl, 1,1-Dimethylpropyl. Vorzugsweise sind R³, R⁵, R⁶, R⁸ gleich -H.

Weitere bevorzugte Diphenylselenoxide weisen als R⁴ und R⁷ iso-Pentyl und als R² und R⁹ gleich Methyl auf und R¹ und R¹⁰ sind beide gleich -Halogen, -H oder -(C₁-C₆)-Alkyl, insbesondere Methyl, Ethyl, Isopropyl, *tert*.-Butyl, iso-Pentyl, 1,1-Dimethylpropyl. Vorzugsweise sind R³, R⁵, R⁶, R⁸ gleich -H.

Weitere bevorzugte Diphenylselenoxide weisen als R², R⁴, R⁷ und R⁹ gleich -Halogen auf, insbesondere sind R² und R⁹ beide gleich -Chlor und R⁴ und R⁷ beide gleich -Fluor oder R², R⁴, R⁷ und R⁹ sind gleich -Fluor, mit R¹ und R¹⁰ beide gleich -Halogen, -H oder -(C₁-C₆)-Alkyl, insbesondere Methyl, Ethyl, Isopropyl, *tert*.-Butyl, iso-Pentyl, 1,1-Dimethylpropyl. Vorzugsweise sind R³, R⁵, R⁶, R⁸ gleich -H.

Weitere bevorzugte Diphenylselenoxide weisen als R², R⁴, R⁷ und R⁹ gleich 1,1-Dimethylpropyl auf mit R¹ und R¹⁰ beide gleich -Halogen, -H oder -(C₁-C₆)-Alkyl, insbesondere Methyl, Ethyl, Isopropyl, *tert*.-Butyl, iso-Pentyl, 1,1-Dimethylpropyl. Vorzugsweise sind R³, R⁵, R⁶, R⁸ gleich -H.

Weitere bevorzugte Diphenylselenoxide weisen als R², R⁴, R⁷ und R⁹ Methyl mit R³ und R⁸ beide gleich -Halogen, -H oder -(C₁-C₆)-Alkyl, insbesondere Methyl, Ethyl, Isopropyl, *tert.-*Butyl, iso-Pentyl, 1,1-Dimethylpropyl. Vorzugsweise sind R¹, R⁵, R⁶, R¹⁰ gleich -H.

Besonders bevorzugt sind R², R³, R⁴, R⁵, R⁶, R⁷, R⁸, R⁹, R¹ und R¹⁰ in Diphenylselenoxiden der Struktur **I** jeweils unabhängig voneinander ausgewählt aus -H und -(C₁-C₁₂)-Alkyl, insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl.

Besonders bevorzugt sind R², R³, R⁴, R⁵, R⁶, R⁷, R⁸, R⁹, R¹ und R¹⁰ in Diphenylselenoxiden der Struktur **I** jeweils unabhängig voneinander ausgewählt aus -(C₁-C₁₂)-Alkyl, insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl.

Besonders bevorzugt sind R², R³, R⁴, R⁵, R⁶, R⁷, R⁸, R⁹, R¹ und R¹⁰ in Diphenylselenoxiden der Struktur **I** jeweils unabhängig voneinander ausgewählt aus -H und -Halogen, ausgewählt aus Chlor, Brom, Fluor, lod.

Besonders bevorzugt sind R², R³, R⁴, R⁵, R⁶, R⁷, R⁸, R⁹, R¹ und R¹⁰ in Diphenylselenoxiden der Struktur **I** gleich -Halogen, ausgewählt aus Chlor, Brom, Fluor und Jod.
Das Diphenylselenoxid der Struktur **I** kann als substituiertes Diphenylselenoxid vorliegen mit R¹ bis R¹⁰ unabhängig ausgewählt aus -H, -(C₁-C₁₂)-Alkyl und/oder -Halogen, vorzugsweise wie vorstehend definiert, wobei mindestens ein R¹ bis R¹⁰ ungleich -H ist.

Das Diphenylselenoxid der Struktur **I** kann als substituiertes Diphenylselenoxid vorliegen mit R², R³, R⁴, R⁷, R⁸ und R⁹ die jeweils unabhängig ausgewählt sein können aus -H, -(C₁-C₁₂)-Alkyl, -Halogen, mit Alkyl linear, verzweigt oder cyclisch,
wobei die Alkylgruppen jeweils unabhängig unsubstituiert sind oder optional kann ein vorgenanntes Alkyl mit mindesten einer -(C₃-C₁₂)-Cycloalkylgruppe substituiert sein, wobei R¹, R⁵, R⁶ und R¹⁰ jeweils unabhängig ausgewählt sind aus -(C₁-C₁₂)-Alkyl und/oder -Halogen, mit Alkyl linear, verzweigt oder cyclisch und mindestens ein Metallatom ausgewählt aus Rh, Ru, Co, Ir. Erfindungsgemäss kann es bevorzugt sein, wenn alle Alkylgruppen unsubstituiert sind.

Das Diphenylselenoxid der Struktur **I** kann als substituiertes Diphenylselenoxid vorliegen mit R², R³, R⁴, R⁷, R⁸ und R⁹ die jeweils unabhängig ausgewählt sein können aus -H, -(C₁-C₁₂)-Alkyl, -Halogen, mit Alkyl linear, verzweigt oder cyclisch,
wobei die Alkylgruppen jeweils unabhängig unsubstituiert sind oder optional kann ein vorgenanntes Alkyl mit mindesten einer -(C₃-C₁₂)-Cycloalkylgruppe substituiert sein, wobei R¹, R⁵, R⁶ und R¹⁰ jeweils unabhängig ausgewählt sind aus -H und/oder -Halogen, und mindestens ein Metallatom ausgewählt aus Rh, Ru, Co, Ir. Erfindungsgemäss kann es bevorzugt sein, wenn alle Alkylgruppen unsubstituiert sind.

Ferner ist Gegenstand der Erfindung die Verwendung eines Diphenylselenoxids der allgemeinen Struktur **I** 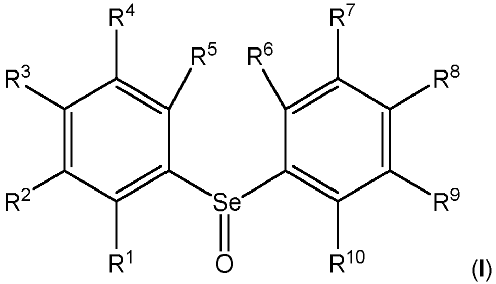 mit R², R³, R⁴, R⁵, R⁶, R⁷, R⁸ und R⁹ jeweils unabhängig ausgewählt aus-H, -(C₁-C₁₂)-Alkyl, -Halogen, mit Alkyl linear, verzweigt oder cyclisch,
wobei die Alkylgruppen jeweils unabhängig unsubstituiert sind oder optional kann ein vorgenanntes Alkyl mit mindesten einer -(C₃-C₁₂)-Cycloalkylgruppe substituiert sein,
mit R¹ und R¹⁰ jeweils unabhängig ausgewählt aus -H, -(C₁-C₁₂)-Alkyl und -Halogen, mit Alkyl linear, verzweigt oder cyclisch,
a) als Ligand in einem Komplex umfassend mindestens ein Metallatom oder
b) mit R¹ und R¹⁰ gleich -Halogen als Zwischenprodukt zur Herstellung von Liganden. Dabei entsprechen die Strukturen des Diphenylselenoxids vorstehenden Definitionen.

Dabei kann die Selenverbindung der Struktur **I** die vorgenannten Substitutionsmuster aufweisen und als unsubstituiertes Diphenylselenid mit R¹ bis R¹⁰ gleich -H oder als substituiertes Diphenylselenid vorliegen mit R¹ bis R¹⁰ wie vorstehend definiert, wobei mindestens ein R¹ bis R¹⁰ ungleich -H ist oder als unsubstituiertes Dipheynlselenoxid mit R¹ bis R¹⁰ gleich -H oder als substituiertes Diphenylselenoxid vorliegen mit R¹ bis R¹⁰ wie vorstehend definiert, wobei mindestens ein R¹ bis R¹⁰ ungleich -H ist, mindestens R¹ oder R¹⁰ ungleich -H ist.

Nach besonders bevorzugten Ausführungsformen ist Gegenstand der Erfindung die Verwendung eines Diphenylselenoxids der allgemeinen Struktur **I**
a) mit R², R³, R⁴, R⁵, R⁶, R⁷, R⁸ und R⁹ jeweils unabhängig ausgewählt aus -H, -(C₁-C₁₂)-Alkyl, -Halogen, mit Alkyl linear, verzweigt oder cyclisch,
   mit R¹, R¹⁰ gleich -H und/oder -(C₁-C₁₂)-Alkyl, mit Alkyl linear, verzweigt oder cyclisch zur Katalyse einer Hydroformylierungsreaktion, oder
b) mit R², R³, R⁴, R⁵, R⁶, R⁷, R⁸ und R⁹ jeweils unabhängig ausgewählt aus -H, -(C₁-C₁₂)-Alkyl, -Halogen, mit Alkyl linear, verzweigt oder cyclisch,
   mit R¹ und R¹⁰ gleich -Halogen als Zwischenprodukt zur Herstellung von Liganden.

Ebenso ist Gegenstand der Erfindung ein Verfahren umfassend die Verfahrensschritte
(i) Vorlegen mindestens eines Olefins,
(ii) Zugabe eines Komplexes umfassend
   - mindestens ein Diphenylselenoxid, welches eine allgemeine Struktur **I** aufweist 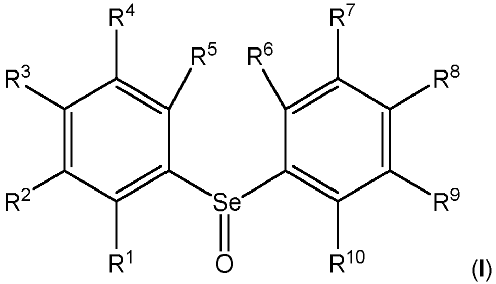 mit R², R³, R⁴, R⁵, R⁶, R⁷, R⁸ und R⁹ jeweils unabhängig ausgewählt aus-H, -(C₁-C₁₂)-Alkyl, -Halogen, mit Alkyl linear, verzweigt oder cyclisch, wobei die Alkylgruppen jeweils unabhängig unsubstituiert sind oder optional kann ein vorgenanntes Alkyl mit mindesten einer -(C₃-C₁₂)-Cycloalkylgruppe substituiert sein,
      und mit R¹ und R¹⁰ jeweils unabhängig gleich -H, -(C₁-C₁₂)-Alkyl und/oder Halogen, mit Alkyl linear, verzweigt oder cyclisch, wobei die Alkylgruppen jeweils unabhängig unsubstituiert sind oder optional kann ein vorgenanntes Alkyl mit mindestens einer -(C₃-C₁₂)-Cycloalkylgruppe substituiert sein, und
   - mindestens ein Metallatom ausgewählt aus Rh, Ru, Co, Ir, insbesondere Rh, Ir, Ru, bevorzugt Rh, oder
      mindestens ein Diphenylselenoxid der allgemeinen Struktur **I**, wie vorstehend beschrieben, und einer Substanz, welche ein Metallatom ausgewählt aus Rh, Ru, Co, Ir aufweist,
(iii) Zuführen von H₂ und CO,
(iv) Erwärmen des Reaktionsgemisches,
wobei das Olefin zu einem Aldehyd umgesetzt wird.

Die Verfahrensschritte (i), (ii), (iii) und (iv) können alternativ in einer beliebigen Reihenfolge durchgeführt werden. Bevorzugt kann in dem Verfahren ein Komplex eingesetzt werden umfassend ein Diphenylselenoxid der Struktur **I**, **Ia**, **Ib**, **Ib***, **Ic** oder **Id** oder es können ein entsprechendes Diphenylselenoxid der Struktur **I**, **Ia**, **Ib**, **Ib***, **Ic** oder **Id** und eine Substanz, welche ein vorgenanntes Metallatom aufweist eingesetzt werden.

Gegebenenfalls kann noch eine weitere Flüssigkeit als Lösungsmittel eingesetzt werden.

Dabei kann vorzugsweise mit den erfindungsgemässen Verbindungen der Strukturen **I**, **Ia**, **Ib**, **Ib*, Ic** oder **Id** in einer Hydroformylierung gemäß vorstehender Verwendung oder vorstehendem Verfahren eine Ausbeute von größer gleich 80 %, insbesondere 85 % und/oder eine n-Regioselektivität von größer 20 %, insbesondere größer gleich 25 % erzielt werden, wobei in Struktur **I** R¹ und R¹⁰ jeweils unabhängig ausgewählt aus -H, -(C₁-C₁₂)-Alkyl und Halogen, mit Alkyl linear, verzweigt oder cyclisch sind.

Nachfolgend wird die Erfindung näher an Beispielen erläutert, ohne die Erfindung auf die Ausführungsbeispiele zu beschränken.

### Allgemeine Methoden

### Lösungsmittel und Reagenzien

Alle Reaktionen mit feuchtigkeits- und/oder sauerstoffempfindlichen Substanzen wurden in ausgeheizten Apparaturen unter Argonatmosphäre durchgeführt. Lösungsmittel zur Extraktion und Säulenchromatographie wurden in folgenden Reinheiten verwendet: Dichlormethan (99.9%, Fa. *Walter,* Art.-Nr. BIE 073107033), Ethylacetat (99.5%, Fa. *Walter,* Art.-Nr. BIE 003917025) und n-Hexan (95%, Fa. *Walter* (*Baker*), Art.-Nr. 8669), n-Heptan (95%, Fa. *Walter (Baker),* Art.-Nr. 8662). Andere Lösungsmittel für Extraktion und Säulenchromatographie waren von technischer Qualität und wurden, sofern nicht anders vermerkt, ohne weitere Reinigung eingesetzt. Trockene Lösungsmittel (abs.) wurden mit einem Pure Solv MD-7 System gereinigt und unter Argonatmosphäre aufbewahrt. Benzylbromid wurde vor dem Gebrauch frisch destilliert (17 mbar/82°C). Deuterierte Lösungsmittel wurden von den angegebenen Trockenmitteln destilliert: Dichlormethan-d₂ (Phosphorpentoxid), Toluol-d₈ (1. KOH; 2. Natrium). Für die Synthesen wurden Chemikalien der Firmen *Sigma Aldrich, Alfa Aesar, Acros Organics, Avantor Performance Materials B.V., Merck KGaA* und *ABCR GmbH* & *Co.* KG verwendet. Diese wurden, sofern nicht anders vermerkt, ohne weitere Reinigung eingesetzt.

### Chromatographische Methoden

Die Säulenchromatographie: Säulenchromatographische Trennungen wurden bei erhöhtem Druck (Flash-Chromatographie) an Silicagel 60 230-400 mesh der Firma *Merck KGaA* (Korngröße: 0.040-0.063 mm) durchgeführt. Die verwendeten Eluentengemische sowie die Volumenverhältnisse v/v sind in den nachfolgenden Vorschriften angegeben. Für die verwendeten Eluenten gelten folgende Abkürzungen: DCM (Dichlormethan), EE (Ethylacetat), H (n-Hexan), sowie Tol (Toluol).

Filtration: Filtrationen zur Abtrennung von entstandenen Feststoffen wurden mit einer G4-Fritte (Porenweite: 10-16 µm) durchgeführt.

### Analytik

¹H-NMR-Spektroskopie: Die ¹H-NMR-Spektren wurden mit dem Modell *AV 300* (300 MHz) sowie mit dem Modell *Fourier 300* (300 MHz) der Firma *Bruker* aufgenommen. Die chemischen Verschiebungen sind in Einheiten der δ-Skala angegeben. Die Restprotonensignale der Lösungsmittel (Dichlormethan-d₂: δ = 5.32 ppm, Toluol-d₈: δ = 7.09; 7.00; 6.98; 2.09 ppm) dienten dabei als Standard.

¹³C-NMR-Spektroskopie: Die ¹³C-NMR-Spektren wurden mit den Modellen *AV 300* (75 MHz) und *Fourier 300* (75 MHz) der Firma *Bruker* aufgenommen. Als interner Standard diente das Signal des Lösungsmittels (Dichlormethan-d₂: δ = 54.0 ppm, Toluol-d₈: δ = 137.9; 129.2; 128.3; 125.5; 20.4 ppm) wobei die chemischen Verschiebungen den ¹H-breitbandentkoppelten Spektren entnommen wurden.

⁷⁷Se-NMR-Spektroskopie: Die ⁷⁷Se-NMR-Spektren wurden mit dem *AV 300* (57 MHz) der Firma *Bruker aufgenommen.* Die Spektren wurden ¹H-breitbandentkoppelt vermessen. Die chemischen Verschiebungen sind in ppm angegeben.

Massenspektrometrie: EI-Massespektren wurden am Gerät *Finnigan MA T 95-XP* der Firma *Thermo Electron* sowie ESI-TOF-Massespektren mit dem Modell *6210 Time-of-Flight LC*/*MS* der Firma *Agilent* aufgenommen.

### Autoklaven-Versuche der Rhodium-katalysierten Hydroformylierung

Die Hydroformylierung wurde in einem mit Druckkonstanthaltung, Gasflussmessung, Begasungsrührer und Druckpipette ausgestattetem 200 mL-Autoklaven der Fa. *Premex Reactor AG,* Lengau, Schweiz, durchgeführt. Das als Lösungsmittel verwendete Toluol wurde mit einem Pure Solv MD-7 System gereinigt und unter Argon aufbewahrt. Das als Substrat eingesetzte Substrat 1-Octen oder n-Octen (*EVONIK Industries AG,* Octenisomerengemisch aus 1-Octen: 3,3 %; *cis*+*trans*-2-Octen: 48.5%; *cis*+*trans*-3-Octen: 29.2%; *cis*+*trans*-Octen-4: 16.4%; gerüstisomere Octene: 2.6%) wurde mehrere Stunden über Natrium am Rückfluss erhitzt und unter Argon destilliert.
Für die Versuche wurden im Autoklaven unter Argonatmosphäre Lösungen der Katalysatorvorstufe und des Liganden gemischt. Als Katalysatorvorstufe kam [(acac)Rh(COD)] (*Umicore,* acac=Acetylacetonat-Anion; COD=1,5-Cyclooctadien) zum Einsatz. Für Versuche mit einer Konzentration von 100 ppm-m Rhodium wurden 10 mL einer 4.31 mM Lösung in den Autoklaven gegeben. Anschließend wurde die einem Verhältnis L/Rh = 5:1 (bzw. 1:1) entsprechende Masse des Liganden in 10 mL Toluol gelöst und zugemischt. Durch Zugabe von weiterem Toluol wurde das Anfangsvolumen der Katalysatorlösung auf 41.0 ml eingestellt. In eine druckfeste Pipette wurde eingefüllt: 1-Octen bzw. n-Octen (10.70 g). Der Autoklav wurde bei einem Gesamtgasdruck (Synthesegas: *Linde;* H₂ (99.999%): CO (99.997%) = 1:1) von a) 42 bar für einen Enddruck von 50 bar bzw. b) 12 bar für den Enddruck von 20 bar unter Rühren (1500 U/min) auf die jeweils angegebenen Temperaturen aufgeheizt. Nach Erreichen der Reaktionstemperatur wurde der Synthesegasdruck auf a) 48.5 bar für einen Enddruck von 50 bar bzw. b) 19.5 bar für einen Enddruck von 20 bar erhöht und das Edukt mit einem in der Druckpipette eingestellten Überdruck von ca. 3 bar zugepresst. Die Reaktion wurde bei konstantem Druck von jeweils 50 bzw. 20 bar (Nachdruckregler der Fa. *Bronkhorst,* NL) über 4h geführt. Der Autoklav wurde nach Ablauf der Reaktionszeit auf Zimmertemperatur abgekühlt, unter Rühren entspannt und mit Argon gespült. Jeweils 1.0 mL der Reaktionsmischungen wurden unmittelbar nach Abschalten des Rührers entnommen, mit 5.0 mL Pentan verdünnt und gaschromatographisch analysiert: HP 5890 Series II plus, PONA, 50 m x 0.2 mm x 0.5 µm.

### Abkürzungen: ber. = berechnet; gef. = gefunden; RT = Raumtemperatur

Die Verbindung **Ia** (CAS 7304-91-8) ist literaturbekannt. **Ia** wurde hergestellt nach: Canadian Journal of Chemistry, 88, 906-909, 2010.

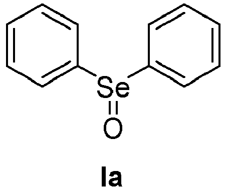

Diphenylselenide können auch entsprechend der europäischen Patentanmeldung EP15168377.8 hergestellt werden. Nach diesem Verfahren können Diphenylselenide hergestellt werden, indem mindestens ein ersten Aren und optional eines zweites Aren in Gegenwart von Selendioxid im Sauren, insbesondere in Gegenwart einer Säure mit einem pKs-Wert im Bereich von 0 bis 5, bei einer definierten Temperatur umgesetzt wird. Ein bevorzugter Temperaturbereich liegt bei 50 bis 120 °C. Die Reaktionsdauer, d.h. die Umsetzung erfolgt vorzugweise über Tage. Die Arene können mit Wasserstoff, Alkyl und/oder Halogen subsituiert sein.

Synthese von Diphenylselenoxiden **I**, insbesondere **Ia.** Analog der folgenden Synthesevorschrift können die Diphenylselenoxide der Struktur **I** gemäß den angegebenen Substituten hergestellt werden.
175 µL (234 mg, 1.00 mmol, 1.0 eq) Diphenylselenid wurde mit 140 mg (1.05 mmol, 1.05 eq) N Chlorsuccinimid umgesetzt. Nach extraktiver Aufarbeitung wurden 235 mg (0.940 mmol, 94%) der Titelverbindung **I** als farbloser Feststoff erhalten.
IR (ATR): (cm-1) = 3044; 3008; 2989; 2941; 1570; 1470; 1437; 1300; 1156; 1069; 1056; 1047; 1017; 993; 915; 850; 820; 733; 686; 611; 481; 442, 1H-NMR (300 MHz, Toluol-d8): δ (ppm) = 7.67-7.51 (m, 4H, Ar-CH); 7.16-6.87 (m, 6H, Ar-CH); 13C-NMR (75 MHz, Toluol-d8): δ (ppm) = 145.1; 130.5; 129.3; 126.0; 77Se-NMR (57 MHz, Toluol-d8): δ (ppm) = 851.0; HR-MS (ESI-TOF): ber. für C12H11OSe ([M+H]+): 250.99700, gef.: 250.99691; ber. für C12H10OSeNa ([M+Na]+): 272.97894, gef.: 272.97888; C12H100Se (249.99 g/mol). Die analytischen Daten stammen mit den Literaturdaten überein.

### Bis(3,5-dimethyl-2-hydroxyphenyl)selen

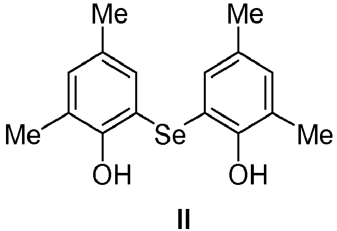

In einem 250 mL Rundkolben wurden 49.9 g Selendioxid (413 mmol) in 100 mL Pyridin mit Hilfe eines Ölbades auf 55 °C erwärmt. Anschließen wurden 25 mL 2,4-Dimethylphenol (206 mmol) hinzugegeben und die Temperatur für siebeneinhalb Stunden gehalten. Nach Beendigung der Reaktion wurde das Gemisch mit 400 mL Ethylacetat verdünnt und filtriert. Die organische Phase wurde mit Wasser gewaschen und über Magnesiumsulfat getrocknet. Das Pyridin wurde destillativ entfernt und der Rückstand erneut in Ethylacetat gelöst und mit 10%iger Salzsäure und Wasser gewaschen um Reste von Pyridin zu entfernen. Die organische Phase wurde mit Magnesiumsulfat getrocknet und unter vermindertem Druck vom Lösungsmittel befreit. Das so erhaltene Rohprodukt wurde in 400 mL Cyclohexan unter Rückfluss erhitzt. Nach Abkühlung auf Raumtemperatur kristallisierte das Produkt. Nach einem Tag wurde das Produkt abfiltriert, das Filtrat auf die Hälfte eingeengt und bei 4 °C erneut zur Kristallisation gebracht. Es wurden 18.56 g, 58 mmol (56%) feine, leicht gelbe Platten des Produktes erhalten
mₚ= 120.1 °C (Rekristallisation aus Cyclohexan).
¹H NMR (400 MHz, CDCl₃) δ= 7.11-7.12 (m, 2H), 6.90-6.92 (m, 2H), 5.95 (br, 2H, OH), 2.23 (s, 6H), 2.19 (s, 6H); ¹³C NMR (100 MHz, CDCl₃) δ= 152.04, 133.35, 133.30, 130.67, 124.42, 115.31, 20.45, 16.69; ⁷⁷Se NMR (76 MHz, CDCl₃) δ= 164.91; HRMS für C₁₆H₁₈O₂⁸⁰Se (ESI+) [M+Na⁺]: berechnet: 345,0370, gefunden: 445.0363;
Elementaranalyse für C₁₆H₁₈O₂Se: berechnet: C: 59.82%, H: 5.65%, gefunden: C: 59.69%, H: 5.76%.

### Katalyse - Hydroformylierung

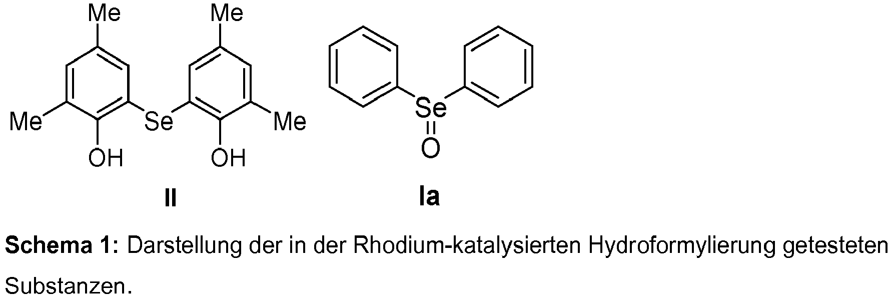

**Tabelle 1: Darstellung der Katalyse-Experimente unter der Verwendung von Organoselenverbindungen.**

| **Eintrag** | **Ligand** | **Olefin /LM** | **Verhältnis Rh/Ligand/Olefin** | **p [bar]** | **T [°C]** | **t [h]** | **A [%]** | **S [%]** |
|---|---|---|---|---|---|---|---|---|
| 1 | **II** | n-Octen / Toluol | 1:1:2197 (100 ppm Rh) | 50 | 120 | 4 | 9.5 | 33.2 |
| 3* | **Ia** | n-Octen / Toluol | 1:1:2205 (40 ppm Rh) | 50 | 120 | 4 | 89.5 | 28.1 |

| | | | | | | | | |
|---|---|---|---|---|---|---|---|---|
| ***Erläuterungen zu Tabelle 1**: p = Druck, T = Temperatur, t = Zeit, A = Ausbeute;* *S = n-Regioselektivität.* * *= erfindungsgemäße Beispiele* | | | | | | | | |

Die Rhodium-katalysierte Hydroformylierung unter Verwendung von Verbindung II (Eintrag 2) führte zu einer Ausbeute von 9.5% (Erhalt von 90.5% Restolefin) und einer n-Regioselektivität von 33.2%.

Die Verwendung von ungeschützten Selenodiphenolen **II**, also solchen mit zwei freien OH-Gruppen, in der Hydroformylierung führt also zu einer Inhibierung.

Durch Verwendung des Diphenylselenoxids **Ia** konnte eine hohe Ausbeute von 89.5% in der Hydroformylierung mit n-Octen verzeichnet werden.
Die Katalyse-Versuche 2) und 3) verdeutlichen den erfolgreichen Einsatz des Diphenylselenoxids in der Rhodium-katalysierten Hydroformylierung. Somit wurde die Aufgabe erfüllt.

## Patentansprüche

1. Komplex umfassend
- mindestens ein Diphenylselenoxid der allgemeinen Struktur (I) 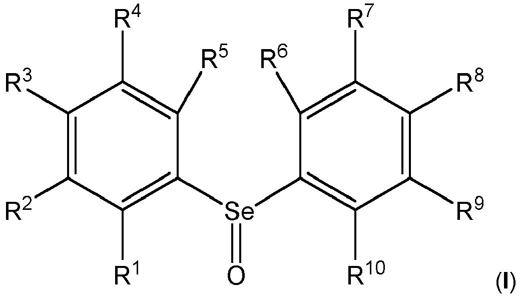 mit R², R³, R⁴, R⁵, R⁶, R⁷, R⁸ und R⁹ jeweils unabhängig ausgewählt aus -H, -(C₁-C₁₂)-Alkyl, -Halogen, mit Alkyl linear, verzweigt oder cyclisch, optional sind die Alkylgruppen mit mindestens einer -(C₃-C₁₂)-Cycloalkylgruppe substituiert,
mit R¹und R¹⁰ jeweils unabhängig ausgewählt aus -H, -(C₁-C₁₂)-Alkyl und -Halogen, mit Alkyl linear, verzweigt oder cyclisch, und
- mindestens ein Metallatom ausgewählt aus Rh, Ru, Co, Ir.

2. Komplex nach Anspruch 1, umfassend
- mindestens ein Diphenylselenid, welches eine allgemeine Struktur (**Ia**) aufweist

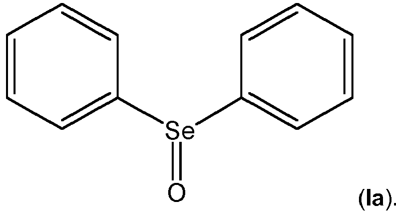

3. Komplex nach Anspruch 1, umfassend
- mindestens ein Diphenylselenid, welches eine allgemeine Struktur **(Ib)** aufweist 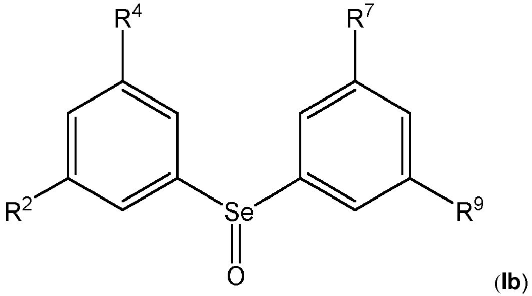 wobei in der Struktur **Ib** R², R⁴, R⁷ und R⁹ jeweils unabhängig ausgewählt sind aus -(C₁-C₁₂)-Alkyl.

4. Komplex nach Anspruch 1, umfassend
- mindestens ein Diphenylselenid, welches eine allgemeine Struktur **(Ib*)** aufweist

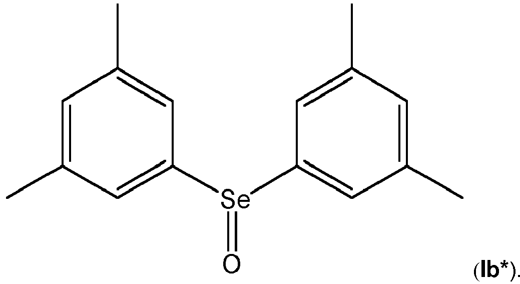

5. Komplex nach Anspruch 1, umfassend
- mindestens ein Diphenylselenid, welches eine allgemeine Struktur (**Ic**) aufweist 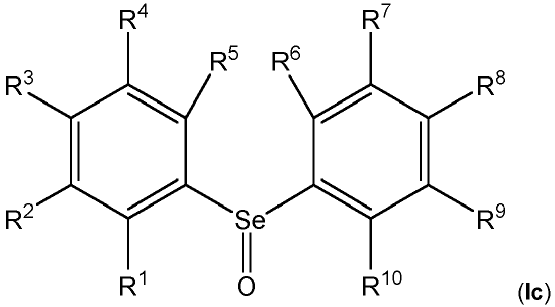 mit R³, R⁵, R⁶ und R⁸ jeweils -H und R², R⁴, R⁷ und R⁹ jeweils unabhängig ausgewählt aus -(C₁-C₁₂)-Alkyl, mit Alkyl linear, verzweigt oder cyclisch,
mit R¹, R¹⁰ jeweils unabhängig ausgewählt aus -H, -(C₁-C₁₂)-Alkyl und -Halogen, mit Alkyl linear, verzweigt oder cyclisch.

6. Komplex nach Anspruch 1, umfassend
- mindestens ein Diphenylselenid, welches eine allgemeine Struktur **(Id)** aufweist 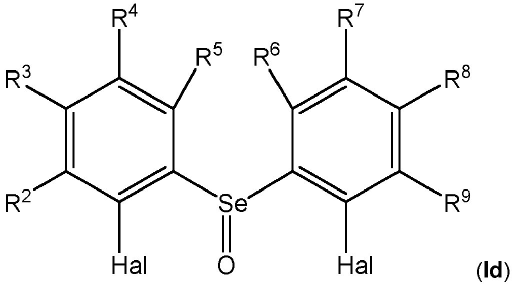 mit R², R³, R⁴, R⁵, R⁶, R⁷, R⁸ und R⁹ jeweils unabhängig ausgewählt aus -H, -(C₁-C₁₂)-Alkyl, und -Halogen.

7. Verwendung eines Diphenylselenoxids der allgemeinen Struktur (I) 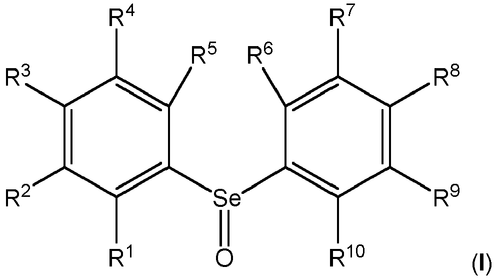 mit R², R³, R⁴, R⁵, R⁶, R⁷, R⁸ und R⁹ jeweils unabhängig ausgewählt aus -H, -(C₁-C₁₂)-Alkyl, -Halogen, mit Alkyl linear, verzweigt oder cyclisch, optional sind die Alkylgruppen mit mindestens einer -(C₃-C₁₂)-Cycloalkylgruppe substituiert,
mit R¹ und R¹⁰ jeweils unabhängig ausgewählt aus -H, -(C₁-C₁₂)-Alkyl und Halogen, mit Alkyl linear, verzweigt oder cyclisch,
a) als Ligand in einem Komplex umfassend mindestens ein Metallatom oder
b) mit R¹ und R¹⁰ gleich Halogen als Zwischenprodukt zur Herstellung von Liganden.

8. Verwendung eines Diphenylselenoxids der allgemeinen Struktur (I) nach Anspruch 1
a) mit R², R³, R⁴, R⁵, R⁶, R⁷, R⁸ und R⁹ jeweils unabhängig ausgewählt aus -H, -(C₁-C₁₂)-Alkyl, -Halogen, mit Alkyl linear, verzweigt oder cyclisch,
mit R¹, R¹⁰ gleich -H oder und -(C₁-C₁₂)-Alkyl, mit Alkyl linear, verzweigt oder cyclisch zur Katalyse einer Hydroformylierungsreaktion, oder
b) mit R², R³, R⁴, R⁵, R⁶, R⁷, R⁸ und R⁹ jeweils unabhängig ausgewählt aus -H, -(C₁-C₁₂)-Alkyl, -Halogen, mit Alkyl linear, verzweigt oder cyclisch,
mit R¹ und R¹⁰ gleich Halogen als Zwischenprodukt zur Herstellung von Liganden.

9. Verfahren umfassend die Verfahrensschritte
(i) Vorlegen mindestens eines Olefins,
(ii) Zugabe eines Komplexes umfassend
- mindestens ein Diphenylselenoxid, welches eine allgemeine Struktur (I) aufweist 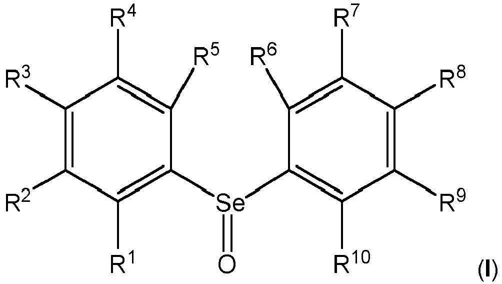 mit R², R³, R⁴, R⁵, R⁶, R⁷, R⁸ und R⁹ jeweils unabhängig ausgewählt aus -H, -(C₁-C₁₂)-Alkyl, -Halogen, mit Alkyl linear, verzweigt oder cyclisch, optional sind die Alkylgruppen mit mindestens einer -(C₃-C₁₂)-Cycloalkylgruppe substituiert,
und mit R¹und R¹⁰ jeweils unabhängig gleich -H, -(C₁-C₁₂)-Alkyl und/oder Halogen, mit Alkyl linear, verzweigt oder cyclisch, und
- mindestens ein Metallatom ausgewählt aus Rh, Ru, Co, Ir, oder
mindestens ein Diphenylselenoxid der allgemeinen Struktur **(I),** wie vorstehend beschrieben,
und einer Substanz, welche ein Metallatom ausgewählt aus Rh, Ru, Co, Ir aufweist,
(iii) Zuführen von H₂ und CO,
(iv) Erwärmen des Reaktionsgemisches,
wobei das Olefin zu einem Aldehyd umgesetzt wird.

## Geänderte Patentansprüche

### Geänderte Patentansprüche gemäss Regel 137(2) EPÜ.

1. Verfahren umfassend die Verfahrensschritte
(i) Vorlegen mindestens eines Olefins,
(ii) Zugabe eines Komplexes umfassend
- mindestens ein Diphenylselenoxid, welches eine Struktur (**Ia**) aufweist 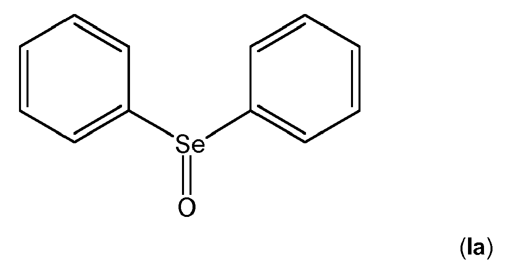 und
- mindestens ein Metallatom ausgewählt aus Rh, Ru, Co, Ir, oder
einer Substanz, welche ein Metallatom ausgewählt aus Rh, Ru, Co, Ir aufweist,
(iii) Zuführen von H₂ und CO,
(iv) Erwärmen des Reaktionsgemisches,
wobei das Olefin zu einem Aldehyd umgesetzt wird.
